(19) Europäisches Patentamt
European Patent Office
Office européen des brevets

(11) Veröffentlichungsnummer : **0 444 586 B1**

# (12) EUROPÄISCHE PATENTSCHRIFT

(45) Veröffentlichungstag der Patentschrift : **27.10.93 Patentblatt 93/43**

(51) Int. Cl.$^5$ : **C07C 49/633,** C07C 35/32, C07C 211/60, A61K 7/00

(21) Anmeldenummer : **91102788.6**

(22) Anmeldetag : **26.02.91**

## (54) Bicyclisches, polysubstituiertes Keton.

(30) Priorität : **02.03.90 CH 663/90**

(43) Veröffentlichungstag der Anmeldung : **04.09.91 Patentblatt 91/36**

(45) Bekanntmachung des Hinweises auf die Patenterteilung : **27.10.93 Patentblatt 93/43**

(84) Benannte Vertragsstaaten : **CH DE ES FR GB IT LI NL**

(56) Entgegenhaltungen :
**EP-A- 0 115 274**
**CH-A- 595 311**
**DE-A- 2 716 537**
**US-A- 3 773 836**
**W. Theilheimer: "Synthetic Methods of Organic Chemistry", vol. 9, 1955, S. Karger, Basel, CH**

(73) Patentinhaber : **GIVAUDAN-ROURE (INTERNATIONAL) S.A.**
**CH-1214 Vernier-Genève (CH)**

(72) Erfinder : **Bachmann, Jean-Pierre**
**Holzmoosrütisteig**
**CH-8820 Wädenswil (CH)**
Erfinder : **Helmlinger, Daniel, Dr.**
**Tichelrütistrasse 18**
**CH-8044 Gockhausen (CH)**
Erfinder : **Pesaro, Mario, Dr.**
**Wiesliacher 55**
**CH-8053 Zürich (CH)**

(74) Vertreter : **Urech, Peter, Dr. et al**
**Grenzacherstrasse 124 Postfach 3255**
**CH-4002 Basel (CH)**

EP 0 444 586 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft einen neuen Riechstoff.

Es handelt sich dabei um das 1,1,2,3,3,6-Hexamethyl-4,5,6,7-tetrahydro -5-indanon, also die Verbindung der Formel

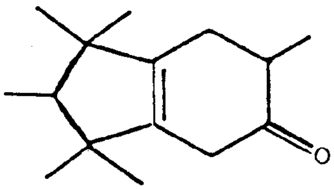

I

Die Verbindung I kann als Gemisch der zwei möglichen Racemate

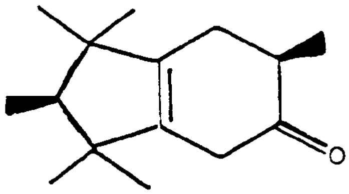

und

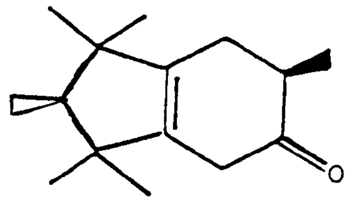

Ia

Ib

oder als eines dieser Racemate vorliegen.

Die Erfindung betrifft ferner ein Verfahren zur Herstellung von I.

Dieses Verfahren ist dadurch gekennzeichnet, dass man 1,1,2,3,3,6-Hexamethyl -4,7-dihydroindan hydroboriert und das Hydroborierungsprodukt zu 1,1,2,3,3,6-Hexamethyl-4,5,6,7-tetrahydro -5-indanon oxydiert, oder dass man 5-Nitro-1,1,2,3,3,6-hexamethylindan reduktiv alkyliert, insbesondere methyliert, und das so erhaltene 5-Dialkylamino-1,1,2,3,3,6-hexamethylindan durch Reduktion des Benzolringes und Hydrolyse des erhaltenen Enamins in 1,1,2,3,3,6-Hexamethyl-4,5,6,7-tetrahydro -5-indanon überführt.

Bei der Hydroborierung von II entsteht durch Anlagerung von Boran an die 5,6-Doppelbindung des Ausgangsmaterials der Formel

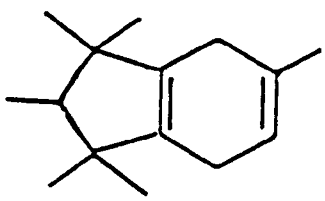

II

ein Organoboran.

Dieses Organoboran kann nun entweder direkt zum Keton I, oder über eine alkoholische Zwischenstufe, nämlich die Verbindung der Formel

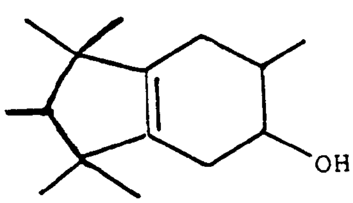

III

zu I oxidiert werden.

Die untenstehende Tabelle I gibt eine detaillierte Uebersicht über die Herstellung der neuen Verbindung I gemäss den beiden Verfahrensvarianten sowie über einen zweckmässigen Zugang zum entsprechenden Ausgangsmaterial II:

Tabelle 1

| Verfahrensschritt | Reaktionstyp | Mittel | Lösungsmittel | Temperatur |
|---|---|---|---|---|
| II→Organoboran | Hydroborierung (1) | $B_2H_6$ | Aether, z.B. Diäthyläther, Tetrahydrofuran, Diglym, etc. | |
| Organoboran →I | Oxidation | $Cr_2O_7^{2-}/H^+$ ($H_2SO_4$) | | -20- +30°C, insb. Raumtemperatur |
| Organoboran→III | Oxidation | $H_2O_2/OH^-$ (NaOH) | | 0-50°C, insb. 0-25°C |
| III→I | Oxidation Alkohol→Keton (2) | insb. $Cr^{6+}$-Salze z.B. Jones-Reagens (4) | Aceton | 0-50°C, insb. 0 - 25°C |
| Herstellung von II | Reduktion (3) | (3) | (3) | (3) |

(1) siehe z.B. Brown, Hydroboration, W.A. Benjamin Inc. N.Y. 1962; Boran-Lösungen in Tetrahydrofuran sind handelsüblich, man kann sie aber auch leicht herstellen, z.B. durch Reaktion von Natriumborhydrid mit Bortrifluoridätherat.

(2) siehe z.B. "Oxidation", Vol. 1, Marcel Bekker Inc. N.Y. (1969) edited by R.L. Augustine, Seiten 56-81.

(3) zweckmässigerweise Reduktion von 1,1,2,3,3,6-Hexamethyl -indan, siehe z.B. die Europäische Patentpublikation Nr. 115 274 vom 8. August 1984.

(4) zweckmässigerweise hergestellt durch Lösen von Chromtrioxid in konzentrierter Schwefelsäure und Verdünnen mit Wasser: J. Chem. Soc., 457 (1953).

Im Falle der reduktiven Alkylierung des Benzolderivates

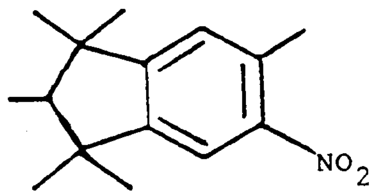

IV

verwendet man vorzugsweise
zunächst das System $H_2$/Pd/Kohle als Reduktionsmittel für die Nitrogruppe auf die Aminostufe (siehe Org. Synthesis, Coll. Volume 5, J. Wiley and Sons, N.Y., (1973), 552) und hierauf
Formaldehyd/$H_2$/Pd/Kohle als Alkylierungs-, bzw. Methylierungsmitel.

Die Herstellung von I schliesslich erfolgt durch Reduktion des Anilinderivates

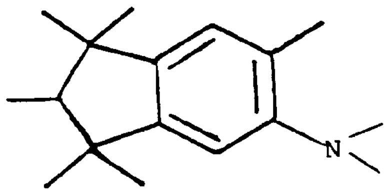

V

nach an sich bekannter Art, z.B. mittels Alkali- bzw. Erdalkalimetallen in flüssigem Ammoniak/Alkohol nach Birch oder mittels Alkali- bzw. Erdalkalimetallen im System Alkylamin/Alkohol nach Benkeser, oder elektrochemisch an Metallkathoden. (Vgl. Houben-Weyl, "Methoden der Organischen Chemie", Band V, Ib (1972) S. 613), gefolgt von Hydrolyse des hydroaromatischen Enamins

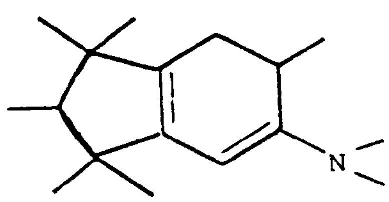

V'

Die Reduktion nach Birch wird bei tiefer Temperatur (z.B. -78° bis -33°C) in flüssigem Ammoniak, das Zusätze eines Alkohols, wie z.B. Aethanol enthält, durchgeführt. Die Reduktion nach Benkeser wird z.B. in Methyl-, Aethylamin, Aethylendiamin, etc. in Gegenwart eines Alkohols wie Aethanol, Isopropanol, Isoamylalkohol usw. durchgeführt.

Als Metalle eignen sich besonders Natrium und Lithium. Ein Zusatz von Diäthyläther oder Tetrahydrofuran begünstigt die Löslichkeit des aromatischen Kohlenwasserstoffes V.

Die Hydrolyse des nach der Reduktion des Benzolringes vorliegenden Enamins zum Keton I wird zweckmässigerweise wässrig sauer, unter milden Bedingungen durchgeführt. Als Säure kann eine anorganische oder auch eine organische Säure dienen. Beispiele sind Salzsäure, Schwefelsäure, Oxalsäure, etc. Man arbeitet vorzugsweise bei Raumtemperatur, doch kommen auch höhere (bis ca. 100°C) oder tiefere (bis ca. 0°C) in Frage.

Die Erfindung betrifft auch die Verwendung der Verbindung I, eines Moschuskörpers, als Riechstoff.

Die Verbindung I zeichnet sich durch kräftige, diffusive und sehr natürlich-warme Noten in Richtung Moschus, mit fruchtigen, sandelholzartigen und animalischen Geruchsaspekten aus.

Die Verbindung der Formel I eignet sich aufgrund ihrer natürlichen Geruchsnoten insbesondere zur Modifizierung von bekannten Kompositionen. Hervorgehoben werden soll nun insbesondere ihre ausserordentliche Geruchsstärke: Der Geruchsschwellenwert beträgt 0,67 ng/l; der Geruchswert 123,900. Bezüglich Definition von Geruchswert und Geruchsschwellenwert siehe z.B. Ulrich A. Huber, Seifen - Oele - Fette - Wachse 110, Nr. 15 (1984) 448-451. Die entsprechenden Werte - unter denselben Bedingungen gemessen - für das bekannte, strukturell ähnliche 6,7-Dihydro-1,1,2,3,3-pentamethyl -5-(4H)-indanon (1,1,2,3,3-Pentamethyl-4,5,6,7-tetrahydro-5-indanon; US-PS 3 773 836) sind andererseits 2,5 ng/l und 35,500.

Die Verbindung I verbindet sich mit zahlreichen bekannten Riechstoffingredientien natürlichen oder synthetischen Ursprungs, wobei die Palette der natürlichen Rohstoffe sowohl leicht- als auch mittel- und schwer-flüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl, Paraguay, Wermutöl,
- Alkohole, wie Farnesol, Geraniol, Linalool, Nerol, Phenyläthylalkohol, Rhodinol, Zimtalkohol,
- Aldehyde, wie Citral, Helional®, α-Hexylzimtaldehyd, Hydroxycitronellal, Lilial® (p-tert.Butyl-α-methyl-dihydrozimtaldehyd), Methylnonylacetaldehyd,
- Ketone, wie Allyljonon, α-Jonon, β-Jonon, Isoraldein (Isomethyl-α-ionon), Methyljonon,
- Ester, wie Allyl-phenoxyacetat, Benzyl-salicylat, Cinnamylpropionat, Citronellyl-acetat, Citronellyl-äthoxalat (Citronellyl . O - CO -CO . $OC_2H_5$), Decyl-acetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat,
- Lactone, wie γ-Undecalacton,
- verschiedene, in der Parfümerie oft benützte Komponenten, wie Ketonmoschus, Indol, p-Menthan-8-thiol-3-on, Methyleugenol.

Bemerkenswert ist ferner die Art und Weise, wie die Verbindung 1 die Geruchsnoten bekannter Kompositionen abrunden und harmonisiert, ohne aber in unangenehmer Weise zu dominieren. So unterstreicht sie z.B. in Parfümbasen mit Tee- und Grün-Charakter die weiche und blumige Note, und in Rosenbasen wird der gesuchte Charakter der schweren und süssen bulgarischen Rose unterstrichen.

Die Verbindung der Formel I (bzw. deren Gemische) lässt sich in weiten Grenzen einsetzen, die beispielsweise von 0,1 (Detergentien) - 5% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 0,2 und 2%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindung I kann demgemäss bei der Herstellung von Kompositionen und - wie obige Zusammenstellung zeigt - unter Verwendung einer breiten Palette bekannter Riechstoffe bzw. Riechstoffgemische, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Beispiel 1

Eine Lösung von 201,3 g eines Gemisches von 60% 1,1,2,3,3,6-Hexamethyl-4,7-dihydro-indan und 19% 1-Aethyl-1,3,3,6-tetramethyl-4,7-dihydro-indan in 700 ml Tetrahydrofuran wird mit 21,8 g fein pulverisiertem Natriumborhydrid versetzt. Dazu wird eine Lösung von 97,4 ml Bortrifluoridäthylätherat (ca. 48% $BF_3$) in 155 ml Tetrahydrofuran gegeben und 2 Stunden bei Raumtemperatur gerührt. Bei +5°C werden innert 15 Minuten 130 ml Wasser, darauf innert 15 Minuten 260 ml 5N NaOH und zuletzt innert 45 Minuten 260 ml 30-prozentiges Wasserstoffperoxid zugetropft. Nach 2 Stunden Rühren bei 50°C wird in Hexan aufgenommen und mit Wasser neutral gewaschen. Nach dem Trocknen der organischen Phase über $MgSO_4$ und Einengen am Rotationsverdampfer verbleiben 215 g farbloses Oel, ein Gemisch von 58% 1,1,2,3,3,6-Hexamethyl-4,5,6,7-tetrahydro -5-indanol und 18% 1-Aethyl-1,3,3,6-tetramethyl-4,5,6,7-tetrahydro -5-indanol. Dieses wird in 1,48 1 Aceton gelöst: bei 0°C werden 365 ml Jones-Reagens so zugetropft, dass die Innentemperatur +6°C nicht übersteigt (ca. 50 Minuten). Nach weiteren 15 Minuten nimmt man in Hexan auf und wäscht mit Wasser neutral; die wässrigen Phasen werden mit Hexan extrahiert und die organische Phase wird über $MgSO_4$ getrocknet. Es verbleiben nach dem Einengen 204 g Oel, welche über eine kleine Vigreuxkolonne schnell-destilliert werden:

Fraktionen:

(1) 72-80°C/0,2 mbar: 5,7 g
(2) 80-103°C/0,2 mbar: 164,0 g
(3) Rückstand: 31,3 g

Die Fraktion (2) wird über eine 30 cm-Widmerkolonne fein-destilliert:

Fraktionen:

(1) 45-62°C/0,05 mbar; $n_D^{20}$ 1,4771-1,4878: 31,1 g

(2) 62-76°C/0,05 mbar; $n_D^{20}$ 1,4878-1,4875: 28,3 g

(1) 76-83°C/0,05 mbar; $n_D^{20}$ 1,4875-1,4884: 86,9 g

(1) 83-84°C/0,05 mbar; $n_D^{20}$ 1,4884 u. >: 8,7 g

Rückstand: 7,1 g

Die Fraktion (3), bestehend aus 75% 1,1,2,3,3,6-Hexamethyl-4,5,6,7-tetrahydro-5-indanon und 25% 1-Aethyl-1,3,3,6-tetramethyl-4,5,6,7-tetrahydro-5-indanon stellt die sogenannte olfaktische Qualität dar; $n_D^{20}$ = 1,4872. IR($CHCl_3$): 1705 $cm^{-1}$

$^1$H-NMR (400 MHz, $CDCl_3$): δ(ppm) 0,77(t); 0,83 (s); 0,89 (s); 0,90 (s); 0,90 (d, J=7 Hz); 0,93 (s); 0,94 (s); 0,96 (s); 0,99 (s); 1,00 (s); 1,04 (2s); 1,08 (2s); 1,115 (d, J=7 Hz); 1,12 (d, J=7 Hz): total 16,5 H.

MS (m/e): 220 ($M^+$), 205, 191, 177, 163, 149, 135, 121, 105, 91,

Geruch: moschusartig, holzig, fichtennadelartig.

Falls gewünscht, kann nun, z.B. mittels präparativer Gaschromatographie, eine Isomerentrennung vorgenommen werden.

Beispiel 2

65,0 g 5-Nitro-1,1,2,3,3,6-Hexamethylindan werden in 580 ml Aethanol aufgeschlämmt und mit 6,5 g 5% Palladium auf Kohle versetzt. Die Luft wird nach Evakuieren mit Stickstoff, und dieser hierauf mit Wasserstoff ersetzt. Man hydriert darauf unter leichter Wasserkühlung bei 22-27°C Innentemperatur. Nach 24 Stunden werden 57,9 ml ca. 36%-ige, wässrige Formaldehydlösung hinzugespritzt. Die Hydrierung setzt ohne Verzug ein. Innert 24 Stunden werden 1,79 Aequivalente $H_2$ aufgenommen. Danach wird der Katalysator abgenutscht. Man destilliert das Aethanol grösstenteils ab, nimmt in tert. Butylmethyläther auf und wäscht mit Wasser.

Die wässrigen Phasen werden zweimal mit tert. Butylmethyläther ausgezogen und die vereinten organischen Phasen nach Trocknung über $MgSO_4$ am Rotationzverdampfer eingeengt. Nach Trocknung im Hochvakuum verbleiben 62,5 g 5-Dimethylamino-1,1,2,3,3,6-hexamethylindan, welche direkt weiterverarbeitet werden.

125,85 g 5-Dimethylamino-1,1,2,3,3,6-hexamethylindan werden in 210 ml Tetrahydrofuran und 630 ml Aethylendiamin gelöst und mit 152,9 ml Diäthylenglykolmonoäthyläther versetzt. Nun kühlt man mittels eines Isopropanol/Trockeneis-Bades auf eine Innentemperatur von +5°C ab und versetzt unter Rühren innert 5 Minuten mit 7,12 g Lithiumdraht in 2-3 cm langen Stücken. Nach 30 Minuten werden bei 20°C Innentemperatur 152,9 ml Diäthylenglykolmonoäthyläther und 7,12 g Lithiumdraht schnell zugegeben. Nach 30 Minuten werden bei 20°C weitere 76,5 ml Diäthylenglykolmonoäthyläther und 3,56 g Lithiumdraht zugegeben. Nach ca. 1 Stunde giesst man das Reaktionsgemisch auf 0,6 1 tert. Butylmethyläther und Eis. Die organische Phase wird darauf mit Wasser neutral gewaschen, die wässrigen Phasen werden mit tert. Butylmethyläther ausgezogen. Die vereinten organischen Phasen werden mit 170 ml Wasser und 104,5 g 65%-iger Schwefelsäure unter leichter Kühlung versetzt. Es wird bei Raumtemperatur während 18 Stunden kräftig gerührt. Die wässrige Phase wird abgetrennt, die organische Phase noch zweimal mit 20% $H_2SO_4$ geschüttelt. Darauf wird noch mit Wasser, mit 4% Sodalösung und mit Wasser neutral gewaschen.

Die wässrigen Phasen werden mit tert. Butylmethyläther ausgezogen, die vereinten organischen Phasen über $MgSO_4$ getrocknet und am Rotavapor eingeengt. Es verbleiben 95,05 g Oel, welche über eine 15 cm Widmerkolonne destilliert werden. Die Fraktionen mit Siedepunkt 81-87°C/0,04 mbar (77,45 g, $n_D^{20}$ 1,4875) stellen die sogenannte olfaktische Qualität an 1,1,2,3,3,6-Hexamethyl-4,5,6,7-tetrahydro-5-indanon dar. Dieses besitzt einen moschusartigen, leicht holzigen, fruchtigen und fichtennadelnartigen Geruch.

Beispiel 3

Blumige Komposition (Richtung Maiglöckchen-Jasmin)

| | |
|---|---|
| Zedernholz destilliert | 50.00 |
| Benzylacetat extra | 80.00 |
| Geraniol extra | 50.00 |
| Isoraldeine 95® Givaudan (alpha-Isomethylionon) | 30.00 |
| Phenyläthylalkohol | 120.00 |
| Hydroxycitronellal | 120.00 |
| Benzylsalicylat | 70.00 |
| Hedione® (Firmenich) | 150.00 |
| α-Hexylzimtaldehyd | 200.00 |
| Cetonial® (Givaudan) (2-Methyl-3-[3',4'-methylendioxybenzol]-propionaldehyd) | 15.00 |
| cis-3-Hexenylsalicylat | 15.00 |
| Evernyl® (Roure S.A.) (Methyl-2,4-dihydroxy-3,5-dimethylbenzoat) | 5.00 |
| Nelkenknospenöl | 8.00 |
| Heliotropin | 8.00 |
| Mandarinenöl | 15.00 |
| Phenylacetaldehyd 85% in Phenyläthylalkohol | 2.00 |
| Undecylaldehyd 1% in Carbitol | 2.00 |
| Dipropylenglykol | 60.00 |

Die Zugabe von 6% 1,1,2,3,3,6-Hexamethyl-4,5,6,7-tetrahydro-5-indanon vermittelt dieser Komposition eine kräftige Ausstrahlung und samtige Weichheit. Ausserdem wird der an und für sich schon natürliche und kosmetische Eindruck dieser Komposition nochmals in aussergewöhnlicher Weise unterstrichen.

**Patentansprüche**

**Patentansprüche für folgende Vertragsstaaten : CH, DE, FR, GB, IT, LI, NL**

1. 1,1,2,3,3,6-Hexamethyl-4,5,6,7-tetrahydro-5-indanon.

2. 1,1,2,3,3,6-Hexamethyl-4,5,6,7-tetrahydro-5-indanol.

3. 5-Dialkylamino-1,1,2,3,3,6-hexamethylindan.

4. 5-Dimethylamino-1,1,2,3,3,6-hexamethylindan.

5. Riechstoffkompositionen, gekennzeichnet durch einen Gehalt an 1,1,2,3,3,6-Hexamethyl-4,5,6,7-tetrahydro -5-indanon.

6. Verfahren zur Herstellung von 1,1,2,3,3,6-Hexamethyl-4,5,6,7-tetrahydro -5-indanon, dadurch gekennzeichnet, dass man 1,1,2,3,3,6-Hexamethyl -4,7-dihydroindan hydroboriert und das Hydroborierungsprodukt zu 1,1,2,3,3,6-Hexamethyl-4,5,6,7-tetrahydro -5-indanon oxydiert, oder dass man 5-Nitro-1,1,2,3,3,6-hexamethylindan reduktiv alkyliert und das so erhaltene 5-Dialkylamino-1,1,2,3,3,6-hexamethylindan durch Reduktion des Benzolringes und Hydrolyse des erhaltenen Enamins in 1,1,2,3,3,6-Hexamethyl-4,5,6,7-tetrahydro -5-indanon überführt.

7. Verwendung von 1,1,2,3,3,6-Hexamethyl-4,5,6,7-tetrahydro -5-indanon als Riechstoff.

**Patentansprüche für folgenden Vertragsstaat : ES**

1. Verfahren zur Herstellung von 1,1,2,3,3,6-Hexamethyl-4,5,6,7-tetrahydro -5-indanon, dadurch gekennzeichnet, dass man 1,2,3,3,6-Hexamethyl -4,7-dihydroindan hydroboriert und das Hydroborierungsprodukt zu 1,1,2,3,3,6-Hexamethyl-4,5,6,7-tetrahydro -5-indanon oxydiert, oder dass man 5-Nitro-1,1,2,3,3,6-hexamethylindan reduktiv alkyliert und das so erhaltene 5-Dialkylamino-1,1,2,3,3,6-hexamethylindan durch Reduktion des Benzolringes und Hydrolyse des erhaltenen Enamins in 1,1,2,3,3,6-Hexamethyl-4,5,6,7-tetrahydro -5-indanon überführt.

2. Riechstoffkompositionen, gekennzeichnet durch einen Gehalt an 1,1,2,3,3,6-Hexamethyl-4,5,6,7-tetrahydro -5-indanon.

3. Verwendung von 1,1,2,3,3,6-Hexamethyl-4,5,6,7-tetrahydro -5-indanon als Riechstoff.

**Claims**

**Claims for the following Contracting States : CH, DE, FR, GB, IT, LI, NL**

1. 1,1,2,3,3,6-Hexamethyl-4,5,6,7-tetrahydro-5-indanone.

2. 1,1,2,3,3,6-Hexamethyl-4,5,6,7-tetrahydro-5-indanol.

3. 5-Dialkylamino-1,1,2,3,3,6-hexamethylindane.

4. 5-Dimethylamino-1,1,2,3,3,6-hexamethylindane.

5. Odorant compositions, characterized in that they contain 1,1,2,3,3,6-hexamethyl-4,5,6,7-tetrahydro-5-indanone.

6. A process for the manufacture 1,1,2,3,3,6-hexamethyl-4,5,6,7-tetrahydro-5-indanone, characterized by hydroborating 1,1,2,3,3,6-hexamethyl-4,7-dihydroindane and oxidizing the hydroboration product to 1,1,2,3,3,6-hexamethyl-4,5,6,7-tetrahydro-5-indanone, or reductively alkylating 5-nitro-1,1,2,3,3,6-hexamethylindane and converting the resulting 5-dialkylamino-1,1,2,3,3,6-hexamethylindane into 1,1,2,3,3,6-hexamethyl-4,5,6,7-tetrahydro-5-indanone by reduction of the benzene ring and hydrolysis of the resulting enamine.

7. The use of 1,1,2,3,3,6-hexamethyl-4,5,6,7-tetrahydro-5-indanone as an odorant.

**Claims for the following Contracting State : ES**

1. A process for the manufacture 1,1,2,3,3,6-hexamethyl-4,5,6,7-tetrahydro-5-indanone, characterized by hydroborating 1,1,2,3,3,6-hexamethyl-4,7-dihydroindane and oxidizing the hydroboration product to 1,1,2,3,3,6-hexamethyl-4,5,6,7-tetrahydro-5-indanone, or reductively alkylating 5-nitro-1,1,2,3,3,6-hexamethylindane and converting the resulting 5-dialkylamino-1,1,2,3,3,6-hexamethylindane into 1,1,2,3,3,6-hexamethyl-4,5,6,7-tetrahydro-5-indanone by reduction of the benzene ring and hydrolysis of the resulting enamine.

2. Odorant compositions, characterized in that they contain 1,1,2,3,3,6-hexamethyl-4,5,6,7-tetrahydro-5-indanone.

3. The use of 1,1,2,3,3,6-hexamethyl-4,5,6,7-tetrahydro-5-indanone as an odorant.

## Revendications

### Revendications pour les Etats contractants suivants : CH, DE, FR, GB, IT, LI, NL

1. 1,1,2,3,3,6-hexaméthyl-4,5,6,7-tétrahydro-5-indanone.

2. 1,1,2,3,3,6-hexaméthyl-4,5,6,7-tétrahydro-5-indanol.

3. 5-dialkylamino-1,1,2,3,3,6-hexaméthylindane.

4. 5-diméthylamino-1,1,2,3,3,6-hexaméthylindane.

5. Compositions odorantes, caractérisées par une teneur en 1,1,2,3,3,6-hexaméthyl-4,5,6,7-tétrahydro-5-indanone.

6. Procédé de préparation de 1,1,2,3,3,6-hexaméthyl-4,5,6,7-tétrahydro-5-indanone, caractérisé en ce qu'on hydrobore le 1,1,2,3,3,6-hexaméthyl-4,7-dihydroindane et qu'on oxyde le produit d'hydroboration en 1,1,2,3,3,6-hexaméthyl-4,5,6,7-tétrahydro-5-indanone ou qu'on alkyle en milieu réducteur le 5-nitro-1,1,2,3,3,6-hexaméthylindane et qu'on transforme le 5-dialkylamino-1,1,2,3,3,6-hexaméthylindane ainsi obtenu par réduction du cycle benzénique et hydrolyse de l'énamine obtenue en 1,1,2,3,3,6-hexaméthyl-4,5,6,7-tétrahydro-5-indanone.

7. Utilisation de 1,1,2,3,3,6-hexaméthyl-4,5,6,7-tétrahydro-5-indanone comme matière odorante.

### Revendications pour l'Etat contractant suivant : ES

1. Procédé de préparation de 1,1,2,3,3,6-hexaméthyl-4,5,6,7-tétrahydro-5-indanone, caractérisé en ce qu'on hydrobore le 1,1,2,3,3,6-hexaméthyl-4,7-dihydroindane et qu'on oxyde le produit d'hydroboration en 1,1,2,3,3,6-hexaméthyl-4,5,6,7-tétrahydro-5-indanone ou qu'on alkyle en milieu réducteur le 5-nitro-1,1,2,3,3,6-hexaméthylindane et qu'on transforme le 5-dialkylamino-1,1,2,3,3,6-hexaméthylindane ainsi obtenu par réduction du cycle benzénique et hydrolyse de l'énamine obtenue en 1,1,2,3,3,6-hexaméthyl-4,5,6,7-tétrahydro-5-indanone

2. Compositions odorantes, caractérisées par une teneur en 1,1,2,3,3,6-hexaméthyl-4,5,6,7-tétrahydro-5-indanone.

3. Utilisation de 1,1,2,3,3,6-hexaméthyl-4,5,6,7-tétrahydro-5-indanone comme matière odorante.